# EUROPÄISCHE PATENTSCHRIFT

(11) **EP 0 661 383 B1**
(45) Veröffentlichungstag und Bekanntmachung des Hinweises auf die Patenterteilung: **21.03.2001**
(21) Anmeldenummer: 94119803.8
(22) Anmeldetag: 15.12.1994
(51) Int. Cl.: C12Q 1/56, G01N 33/86

(54) **Verfahren zur Bestimmung der Thrombozytenaggregation**
Method for the determination of thrombocyte aggregation
Méthode pour la détermination d'agrégation de thrombocytes

(30) Priorität: 30.12.1993 DE 4344919
(43) Veröffentlichungstag der Anmeldung: 05.07.1995
(73) Patentinhaber: Dade Behring Marburg GmbH, 35001 Marburg (DE)
(72) Erfinder: Reers, Martin, Dr., D-35041 Marburg-Michelbach (DE)

(56) Entgegenhaltungen:
- EP-A- 0 336 353
- EP-A- 0 537 490
- US-A- 5 246 832

## Beschreibung

Die Erfindung betrifft ein Verfahren zur Bestimmung der Thrombozytenaggregation in Gegenwart eines Hemmers der Fibrinaggregation, welcher die Bildung eines störenden Fibrinclots verhindert sowie ein Diagnostikum zur Bestimmung der thrombozytenaggregationshemmenden Wirkung von Thrombininhibitoren.

Zur Bestimmung der der thrombozytenaggregationshemmenden Wirkung von Thrombininhibitoren können beispielsweise diagnostische Systeme verwendet werden, die eine Thrombozyten-Suspension wie blutplättchenreiches Plasma ("platelet rich plasma"; PRP) und Thrombin enthalten, wobei das Thrombin eine Aggregation der Thrombozyten auslöst. Die Geschwindigkeit oder das Ausmaß der Aggregation kann mit einer Trübungsmessung, beispielsweise turbidimetrisch oder nephelometrisch bestimmt werden. Eine Zugabe von Thrombin-Inhibitoren verzögert in Abhängigkeit von der zugegebenen Menge die Plättchenaggregation, so daß aus der Trübungsmessung die inhibitorische Wirkung quantifiziert werden kann.

Bei einer z. B. thrombininduzierten Thrombozytenaggregation in plättchenreichem Plasma (PRP) bereitet ein thrombininduzierter Fibrinclot meßtechnische Schwierigkeiten. In den Verfahren des Standes der Technik können nur sehr niedrige Konzentrationen an Thrombin eingesetzt werden, so daß eine maximale Thrombozytenaggregation nicht erreicht werden kann. Aber auch bei niedrigen Thrombinkonzentrationen entstehen nach einer gewissen Zeit störende Fibrinclots. Um dennoch höhere Konzentrationen an Thrombin einsetzen zu können, welche zu einem besseren Meßsignal führen, muß ein aufwendiges Plättchenwaschverfahren durchgeführt werden.

Die Aufgabe vorliegender Erfindung bestand deshalb darin, ein Verfahren bereitzustellen, das die Bestimmung der experimentell induzierten Thrombozytenaggregation und die Bestimmung von Thrombininhibitoren ohne den störenden Einfluß eines Fibrinclots ermöglicht.

Es ist bekannt, daß kurze Peptide beginnend mit der Sequenz Gly-Pro-Arg an Fibrinogen binden (Proc. Natl. Acad. Sci U.S.A., 1978, 75,3085-3089). Weiterhin ist das Peptid Gly-Pro-Arg-Pro in Desaggregationsexperimenten von Thrombozyten in Kombination mit Antiplatelet-Reagenzien (Pharmacology and Experimental Therapeutics, 1991, 259, 1371-1378) verwendet worden. Außerdem ist bekannt, daß Gly-Pro-Arg-Pro-Ala-NH₂ als Fibrinaggregationshemmer in Verfahren zur Bestimmung von F Xllla und von Fibrinogen eingesetzt werden kann.

Überraschenderweise wurde nun gefunden, daß durch Zusatz eines Hemmers der Fibrinaggregation die Bildung eines störenden Fibrinclots auch bei hohen Thrombinkonzentrationen ohne Einfluß auf die experimentell erwünschte Thrombozytenaggregation unterdrückt wird.

Die vorliegende Erfindung betrifft somit ein Verfahren zur qualitativen oder quantitativen Bestimmung der durch Thrombin ausgelösten Thrombozytenaggregation, dadurch gekennzeichnet, daß durch die Anwesenheit eines Hemmers der Fibrinaggregation die Bildung eines störenden Fibrinclots verhindert wird.

Geeignete Hemmer der Fibrinaggregation sind Peptide oder Peptidderivate, insbesondere solche, die in der Struktur analog dem aminoterminalen Ende der humanen α-Fibrinkette sind. Solche Hemmer der Fibrinaggregation werden beispielsweise in A. P. Laudano et al. (1978) Proc. Natl. Acad. Sci. U.S.A., 75, 3085-3089 beschrieben. Es sind diejenigen Peptide bevorzugt, welche die Sequenz Gly-Pro-Arg oder Gly-Pro-Arg-Pro enthalten.

Insbesondere bevorzugt ist das Peptid bzw. Peptidderivat mit der Sequenz Gly-Pro-Arg,

Gly-Pro-Arg-Pro oder Gly-Pro-Arg-Sar.

Weiterhin besonders bevorzugt ist

Gly-Pro-Arg-Pro-Ala-NH2.

Die Erfindung betrifft weiterhin die Verwendung des oben beschriebenen Verfahrens zur Bestimmung der thrombozytenaggregationshemmenden Wirkung von Thrombininhibitoren, dadurch gekennzeichnet, daß der zu bestimmende Thrombininhibitor und der Hemmer der Fibrinaggregation gleichzeitig im Testansatz vorhanden sind. Überraschenderweise können mit diesem Verfahren beispielsweise im Plasma vorhandene Thrombininhibitoren nicht nur nachgewiesen, sondern auch quantifiziert werden. Der qualitative bzw. quantitatve Nachweis ist aber auch in anderen Flüssigkeiten als Plasma mit dem erfindungsgemäßen Verfahren möglich.

Die Erfindung betrifft weiterhin ein Diagnostikum, enthaltend Thrombin und einen Inhibitor der Fibrinaggregation. Ein solches Diagnostikum eignet sich zur Durchführung des erfindungsgemäßen Verfahrens zur qualitativen oder quantitativen Bestimmung der thrombozytenaggregationshemmenden Wirkung von Thrombininhibitoren in Flüssigkeiten, beispielsweise in Plasma.

Im folgenden wird beispielhaft ein Verfahren zur Bestimmung von Inhibitoren der Thrombozytenaggregation, z. B. in einer Pufferlösung oder in Patientenproben beschrieben. Der Test kann in folgenden Schritten durchgeführt werden:
- Vorlage von thrombozytenreichem Plasma (PRP; siehe oben)
- Zusatz von Citratlösung oder thrombozytenarmem Plasma (PPP)
- Zusatz des Fibrinaggregationshemmers, welcher gegebenenfalls in einer Pufferlösung gelöst vorliegt
- Zugabe der zu testenden Probe; der nachzuweisende Inhibitor kann beispielsweise in einer Pufferlösung oder in PPP gelöst bzw. in Plasma vorliegen; es werden unterschiedliche Konzentrationsstufen (Verdünnungsreihe) getestet
- Inkubation über einen Zeitraum von 1 bis 20 Minuten bei ca. 37 °C
- Zugabe eines Induktors der Thrombozytenaggregation, beispielsweise Thrombin
- Messung der Thrombozytenaggregation

Diese Bestimmung benötigt nur kleine Volumina: PRP, vorzugsweise 300 µl; Lösung des Fibrinaggregationshemmers, vorzugsweise 25 µl; Thrombininhibitorlösung, beispielsweise CRC 220 (4-methoxy-2,3,6-trimethylphenylsufonyl-L-aspartyl-D-4-amidinophenyl-alanylpiperidide EP 0 513 543) oder PPP, vorzugsweise 25 µl. Das Gesamtvolumen beträgt 300-1000 µl, vorzugsweise 500 µl. Um ein definiertes Volumen von beispielsweise 500 µl zu erhalten, kann eine Lösung zugesetzt werden, welche aus einer 0,38%igen Citratlösung besteht, die mit isotonischer NaCI-Lösung 10-fach verdünnt worden ist. Vorzugsweise wird die Thrombozytenaggregationsmessung in einer 1 ml Küvette in einem Aggregometer wie beispielsweise APACT (Automated Platelet Aggregation and Coagulation Tracer) der Firma LAbor (Laborgeräte und Analysesysteme Vertriebsgesellschaft mbH, Ewige Weide 7, D-22926 Ahrensburg, Deutschland), bei 37°C, unter Rühren durchgeführt.

PRP kann auf bekannte Weise in einer Citratlösung hergestellt werden. Der Bereich der Thrombozytenzahl liegt zwischen 10⁷ und 10⁹ pro ml, vorzugsweise bei 2x10⁸ pro ml. Die Konzentration der Thrombozyten für eine optimale Aggregationsfähigkeit kann mit Hilfe eines gebräuchlichen Thrombozytenagonisten, beispielsweise Kollagen, ADP oder Thromboxan A₂ bestimmt werden.

Die Konzentration des Fibrinaggregationshemmers liegt im Bereich von 1-20 mM, vorzugsweise 10 mM.

Der Konzentrationsbereich des Thrombins liegt bevorzugt bei 4-7 nM, entsprechend etwa 80 % der maximal durch Thrombin erreichbaren Thrombozytenaggregation.

### Abkürzungen:

- PRP: thrombozytenreiches Plasma
- PPP: thrombozytenarmes Plasma
- Gly: Glycin
- Pro: L-Prolin
- Arg: L-Arginin
- Ala: L-Alanin
- Sar: Sarkosin
- NH₂: Amid
- mM: millimol/Liter
- nM: nanomol/Liter

Die folgenden Beispiele erläutern die Erfindung näher:

### Beispiel 1:

### Bestimmung der Thrombozytenaggregation

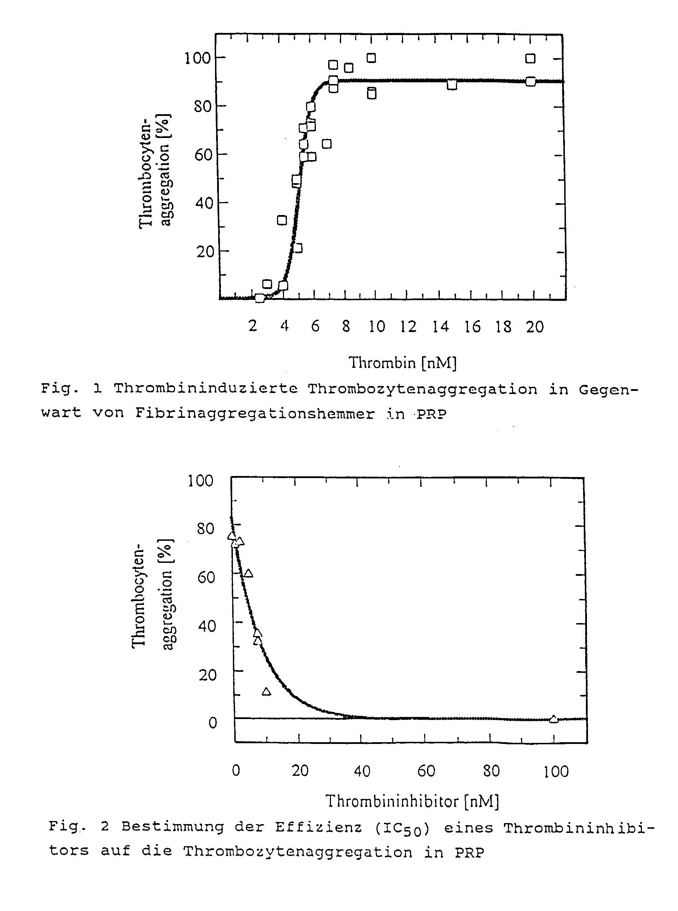

Fig. 1 zeigt die thrombininduzierte Thrombozytenaggregation (Verhältnis aggregierte zu nicht aggregierten Thrombozyten in %) in PRP in Abhängigkeit von der Konzentration an Thrombin in nM in Gegenwart eines Fibrinaggregationshemmers. Auf diese Weise wird erreicht, daß eine deutlich höhere Thrombinkonzentration als beim Stand der Technik eingesetzt werden kann. Eine auf diese Weise gespreizte Bezugskurve trägt wesentlich zu einer präzisen Bestimmung der Thrombocytenaggregation bei.

Wie aus Fig. 1 zu erkennen, nimmt die Thrombozytenaggregation über den Bereich von etwa 10-80 % der maximal erreichbaren Thrombozytenaggregation linear mit der zugesetzten Thrombinkonzentration von 4-7 nM^{.}(0,4-0,7 IE/ml) zu.

### Beispiel 2

### Bereitstellung der Lösung A (Citrat-Lösung)

Es wird folgende Substanz in 100 ml Wasser aufgelöst: 380 mg Tri-natrium-citrat-dihydrat. Die Lösung kann gegebenenfalls auch 500 mg D-(+)-Glucose und 350 mg Albumin enthalten.

### Bereitstellung der Lösung B (PRP)

90 ml frisches Vollblut von Spendern werden in ein Polyethylengefäß gefüllt, das bereits 10 ml einer 3,8 %igen (w/v) Tri-natrium-citrat-dihydrat Lösung enthält. Dieses antikoagulierte Blut wird umgehend bei 200 g für 20 min. zentrifugiert. Der Überstand wird in ein neues Polyethylengefäß überführt und bis zur Verwendung bei 0 °C gelagert.

### Bereitstellung der Lösung C (Fibrinaggregationshemmer)

Es werden folgende Substanzen in 100 ml Wasser aufgelöst: 1 g Rinderserum-Albumin und 10 g Gly-Pro-Arg-Pro-Ala-NH₂. Die Lösung wird bei -20 °C bis zur Verwendung gelagert.

### Bereitstellung der Lösung D (Thrombininhibitor)

Es wird folgende Substanz in 100 ml Wasser aufgelöst: 127,6 mg Thrombininhibitor CRC 220 (4-methoxy-2,3,6-trimethylphenylsulfonyl-L-aspartyl-D-4-amidinophenyl-alanyl-piperidide). Für die Verwendung im Test werden mit Hilfe von isotonischer Kochsalzlösung verdünnte CRC 220 Lösungen bereitgestellt. Alle Lösungen werden frisch angesetzt und auf 0 °C gekühlt.

### Bereitstellung der Lösung E (Thrombin)

4,2 µg humanes α-Thrombin werden in 1 ml Wasser aufgelöst (10 IE/ml). Für die Verwendung im Test werden mit Hilfe von isotonischer Kochsalzlösung verdünnte Thrombin-Lösungen bereitgestellt. Alle Lösungen werden frisch angesetzt und auf 0 °C gekühlt.

### Testausführung

300 µl Lösung B, 100 µl Lösung A, 25 µl Lösung C und 25 µl Lösung D werden in eine 1 ml fassende Meßküvette (1 cm Lichtweg, ständiges Rühren) des Aggregometers APACT (LAbor) pipettiert und bei 37°C für 1 min vorinkubiert. Danach werden 50 µl Lösung E zugegeben und die Lichttransmission in Abhängigkeit von der Zeit aufgezeichnet. Die Messung wird bis zur Konstanz des Signals verfolgt, welche in der Regel nach 5 Minuten erreicht ist. Pro Testansatz werden aufsteigende Konzentrationen an Thrombininhibitor zugesetzt. Mit Kenntnis der zugegebenen Mengen an Thrombininhibitor läßt sich quantitativ beispielsweise ein IC₅₀-Wert für den Thrombininhibitor bestimmen (Fig. 2). Für dieses Beispiel ergibt sich ein IC₅₀-Wert von 10 nM.

## Patentansprüche

1. Verfahren zur qualitativen oder quantitativen Bestimmung der durch Thrombin ausgelösten Thrombozytenaggregation bei Gegenwart von Fibrin, dadurch gekennzeichnet, daß durch die Anwesenheit eines Hemmers der Fibrinaggregation die Bildung eines störenden Fibrinclots verhindert wird.

2. Verfahren nach Anspruch 1, dadurch gekennzeichnet, daß der Hemmer der Fibrinaggregation ein Peptid oder Peptidderivat ist.

3. Verfahren nach Anspruch 2, dadurch gekennzeichnet, daß das Peptid oder Peptidderivat an seinem aminoterminalen Ende eine dem aminoterminalen Ende der humanen α-Fibrinkette analoge Struktur enthält.

4. Verfahren nach Anspruch 3, dadurch gekennzeichnet, daß die Struktur ein Peptid mit der Sequenz Gly-Pro-Arg oder Gly-Pro-Arg-Pro ist.

5. Verfahren nach Anspruch 2, dadurch gekennzeichnet, daß das Peptid oder Peptidderivat die Sequenz Gly-Pro-Arg, Gly-Pro-Arg-Pro, Gly-Pro-Arg-Sar oder Gly-Pro-Arg-Pro-Ala-NH₂ hat.

6. Verfahren gemäß Anspruch 1, dadurch gekennzeichnet, daß Thrombin 4 bis 7 nM eingesetzt wird.

7. Verwendung des Verfahrens nach Anspruch 1 zur qualitativen oder quantitativen Bestimmung der thrombozytenaggregationshemmenden Wirkung von Thrombininhibitoren, dadurch gekennzeichnet, daß der Thrombininhibitor und der Hemmer der Fibrinaggregation gleichzeitig im Testansatz vorhanden sind.

## Claims

1. A method for the qualitative or quantitative determination of platelet aggregation induced by thrombin in the presence of fibrin, wherein the formation of an interfering fibrin clot is prevented by the presence of an inhibitor of fibrin aggregation.

2. The method as claimed in claim 1, wherein the inhibitor of fibrin aggregation is a peptide or peptide derivative.

3. The method as claimed in claim 2, wherein the peptide or peptide derivative contains at its amino-terminal end a structure analogous to the amino-terminal end of the human α fibrin chain.

4. The method as claimed in claim 3, wherein the structure is a peptide having the sequence Gly-Pro-Arg or Gly-Pro-Arg-Pro.

5. The method as claimed in claim 2, wherein the peptide or peptide derivative has the sequence Gly-Pro-Arg, Gly-Pro-Arg-Pro, Gly-Pro-Arg-Sar or Gly-Pro-Arg-Pro-Ala-NH₂.

6. The method as claimed in claim 1, wherein 4 to 7 nM thrombin is employed.

7. The use of the method as claimed in claim 1 for the qualitative or quantitative determination of the platelet aggregation-inhibiting action of thrombin inhibitors, wherein the thrombin inhibitor and the inhibitor of fibrin aggregation are present simultaneously in the assay mixture.

## Revendications

1. Procédé pour la détermination qualitative ou quantitative de l'agrégation des thrombocytes déclenchée par la thrombine, en présence de fibrine, caractérisé en ce que la formation d'un caillot gênant de fibrine est empêchée par la présence d'un inhibiteur de l'agrégation de la fibrine.

2. Procédé selon la revendication 1, caractérisé en ce que l'inhibiteur de l'agrégation de la fibrine est un peptide ou un dérivé de peptide.

3. Procédé selon la revendication 2, caractérisé en ce que le peptide ou dérivé de peptide comporte à son extrémité amino-terminale une structure analogue à l'extrémité amino-terminale de la chaîne α de la fibrine humaine.

4. Procédé selon la revendication 3, caractérisé en ce que la structure est un peptide ayant la séquence Gly-Pro-Arg ou Gly-Pro-Arg-Pro.

5. Procédé selon la revendication 2, caractérisé en ce que le peptide ou dérivé de peptide a la séquence Gly-Pro-Arg, Gly-Pro-Arg-Pro, Gly-Pro-Arg-Sar ou Gly-Pro-Arg-Pro-Ala-NH₂.

6. Procédé selon la revendication 1, caractérisé en ce que l'on utilise de la thrombine 4 à 7 nM.

7. Utilisation du procédé selon la revendication 1, pour la détermination qualitative ou quantitative de l'activité, inhibitrice de l'agrégation des thrombocytes, d'inhibiteurs de thrombine, caractérisée en ce que l'inhibiteur de thrombine et l'inhibiteur de l'agrégation de la fibrine sont présents simultanément dans le mélange d'essai.
